# EUROPEAN PATENT SPECIFICATION

(11) **EP 3 041 456 B1**
(45) Date of publication and mention of the grant of the patent: **04.12.2019**
(21) Application number: 14758619.2
(22) Date of filing: 27.08.2014
(51) Int. Cl.: A61Q 7/00, A61K 8/31, A61K 36/15, A61K 31/015, A61P 17/14

(54) **COMPOSITION AND METHOD FOR INDUCING OR STIMULATING GROWTH OF KERATINOUS FIBRES**
ZUSAMMENSETZUNG UND VERFAHREN ZUR AKTIVIERUNG UND FÖRDERUNG DES WUCHSES VON KERATINFASERN
COMPOSITION ET MÉTHODE POUR INDUIRE OU STIMULER LA CROISSANCE DE FIBRES KÉRATINIQUES

(30) Priority: 27.08.2013 GB 201315231
(43) Date of publication of application: 13.07.2016
(73) Proprietor: Gilloteaux, Jacques, 5330 Assesse (BE)
(72) Inventor: Gilloteaux, Jacques, 5330 Assesse (BE)
(74) Representative: Murgitroyd & Company
(86) International application number: PCT/GB2014/052591
(87) International publication number: WO 2015/028788

(56) References cited:
- EP-A1- 1 336 402
- JP-A- 2004 002 258
- DATABASE GNPD [Online] MINTEL; November 2011 (2011-11), Anonymous: "Anti-Hair Loss Strengthening Serum", XP002731719, Database accession no. 1677606
- DATABASE WPI Week 200926 Thomson Scientific, London, GB; AN 2009-F06369 XP002731720, -& KR 100 873 946 B1 (BIO SPECTRUM INC) 12 December 2008 (2008-12-12)

## Description

### Field of the Invention

The invention relates to a composition and method for inducing or stimulating growth of keratinous fibres. In particular, the invention relates to the use of pinene for inducing or stimulating hair follicle hyperplasia.

### Background to the Invention

Human hair follows a growth cycle having three distinct phases - anagen, catagen and telogen. At any one time, each strand of hair is in a particular phase of development, and neighbouring hairs may be in different stages.

The average rate of hair growth in humans is around 1.25cm per month, and natural hair loss occurs regularly, at around one hundred strands of hair per day.

Increased hair loss can be caused by a number of factors including genetic factors, lifestyle, pregnancy and underlying medical conditions.

There are various treatments to prevent or slow hair loss and promote hair re-growth. Perhaps the most well-known treatment is Minoxidil, which is a vasodilator that may be taken orally or more usually, as a topical treatment to the scalp. Whilst Minoxidil can be an effective treatment for pattern baldness, this requires continuous use and it may not be effective against other causes of hair loss. Further, regrowth may be slow and side effects can include, skin irritation, unwanted hair growth and even an increase in shedding of some hair.

More recently a synthetic molecule has been developed to promote hair growth and increase hair density. The product containing this molecule is marketed under the trade name Stemoxydine®. The active ingredient is diethyl pyrideine-2, 4-dicarboxylate and it is believed to enhance hair density by mimicking hypoxia conditions to stimulate hair follicles.

In order to minimise side effects from the use of synthetic products, it may be desirable to use a naturally-occurring products for promoting hair growth. One example of a herbal product that is said to induce hair growth at a rate comparable to Minoxidil, is leaf sap from the common weed *Eclipta alba.*

Pinene (C₁₀H₁₆) is an organic monoterpene chemical compound, which is present in many natural oils from coniferous trees (e.g. pine resin), some plants and essential oils, such as oil of rosemary and eucalyptus oil.

Two structural isomers of pinene are found in nature: α-pinene and β-pinene.

Both α-pinene and β-pinene exist as enantiomers and are present in nature in a racemic mixture. For example α-pinene has enantiomers (-)-α-pinene and (+)-α-pinene.

Monoterpenes have been used in cosmetic and pharmaceutical preparations, such as anti-inflammatory preparations, skin treatments and hair dyes.

Pinene is most commonly used in cosmetic and pharmaceutical preparations as a perfume.

For example, JPH0267211 (Sasaki Chemical) describes a skin and hair treating agent which includes a citrus oil containing terpene, pinene, limonene, citral, citronellal or turpentine oil.

JP2004300100 (Hoyu KK) also describes a hair dye composition with a cyclic monoterpene component, which may be pinene.

JP60139612 (Lion Corp) describes a hair cosmetic containing an N-acylamino acid and a terpene compound (such as pinene, limonene, myrcene). The cosmetic is said to be capable of providing the hair with lustre without causing greasiness by synergistic effect.

JPH11246369 (YAMAHATSU SANGYO KAISHA) discloses a hair dye with improved penetration of hair. The penetrating agent is said to be obtained by including at least one of monoterpene dimers (e.g. alpha -pinene dimer, camphene dimer, d-limonene dimer or the like).

The use of the alpha-pinene isomer has also been described as a skin treatment for reducing wrinkles. KR100873946 (Bio Spectrum Inc.) describes agents for improving wrinkles on skin comprising alpha-pinene as an active ingredient. The composition is said to contain 0.0001-10.0 wt. % of alpha-pinene as an effective ingredient.

As well as stimulating human keratinous fibres, it may be desirable to stimulate growth of animal coats, for example, for increasing wool production from sheep.

It would be desirable to provide an improved composition and method for inducing or stimulating growth of keratinous fibres.

### Summary of the Invention

One aspect of the invention provides a composition as claimed in Claim 1.

Another aspect of the invention provides a method as claimed in Claim 10.

Also described is a shampoo, lotion, cream, gel, hair styling product, beauty product, skin preparation, pharmaceutical preparation or ointment.

Yet another aspect of the invention provides a use of a composition for increasing the density of the keratinous fibres, as claimed in Claim 9.

The invention provides a composition, for use in inducing or stimulating growth of keratinous fibres for treating human baldness in adult males or caused by anticancer treatments, wherein the composition comprises pinene as an active ingredient.

The active ingredient pinene comprises the α-pinene isomer of pinene.

At least 70% of the active ingredient pinene is the (-) - α enantiomer of pinene.

More preferably, at least 80% of the active ingredient pinene is the (-) - α enantiomer of pinene.

More preferably, at least 90% of the active ingredient pinene is the (-) - α enantiomer of pinene.

In one embodiment, the active ingredient pinene is present as substantially optically pure (-) - α enantiomer of pinene.

Preferably, the composition is substantially free of the β-pinene isomer of pinene

In one embodiment, the composition may be substantially free of the (+)-α enantiomer of pinene.

In one embodiment, the composition may comprise the (-)-α enantiomer of pinene and the (+)-α enantiomer of pinene in a ratio of about 500:1.

The active ingredient pinene may be present in the composition at about 5% (w/w) or above.

The active ingredient pinene is present in the composition at between about 0.5% to about 10% (w/w).

More preferably, the active ingredient pinene may be present in the composition at between about 0.5% to about 5% (w/w).

The composition may further comprise a solvent.

The solvent may comprise an alcohol.

The solvent may comprise acetone or ethanol.

The solvent may comprise isopropyl alcohol.

The solvent may comprise dimethyl sulfoxide (DMSO).

Additionally, or alternatively, the solvent may comprise glycerine, glycerol, or a glycerol based solution.

The composition may consist of pinene as an active ingredient and a solvent.

The keratinous fibres may comprise hair, wool, pelt, coat or fur.

The hair may comprise head hair, eyelashes, facial hair or body hair.

Optionally, the composition is a cosmetic or dermatological composition.

The composition may, further comprise a pharmaceutically or cosmetically acceptable excipient.

Advantageously, the composition is for increasing the density of the keratinous fibres.

Another aspect of the invention provides a non-therapeutic method for inducing or stimulating growth of keratinous fibres comprising the step of administering to a subject an effective amount of a composition comprising pinene as an active ingredient.

The active ingredient pinene comprises the α-pinene isomer of pinene.

At least 70% of the active ingredient pinene is the (-) - α enantiomer of pinene.

Preferably, at least 80% of the active ingredient pinene is the (-) - α enantiomer of pinene.

More preferably, at least 90% of the active ingredient pinene is the (-) - α enantiomer of pinene.

The active ingredient pinene may be present as substantially optically pure (-) - α enantiomer of pinene.

In one embodiment, the composition may comprise the (-)-α enantiomer of pinene and the (+)-α enantiomer of pinene in a ratio of about 500:1.

In one embodiment, the composition may comprise the (-)-α enantiomer of pinene and the (+)-α enantiomer of pinene in a ratio of about 500:50.

The composition may be substantially free of the (+)-α enantiomer of pinene.

The active ingredient pinene may be present in the composition at about 5% (w/w) or above.

The active ingredient pinene is present in the composition at between about 0.5% to about 10% (w/w).

The subject may be a human or animal subject.

The step of administering may comprise topical, oral of injectable administration of the composition.

Administration may be to the scalp and/or hair of a human subject.

The composition may be administered twice weekly for at least 5 weeks.

A shampoo, lotion, cream, gel, hair styling product, beauty product, skin preparation, pharmaceutical preparation or ointment comprising the composition is described.

The use of pinene for inducing or stimulating growth of keratinous fibres is also described.

Preferably, the use of pinene is the use of the α-pinene isomer of pinene, substantially in the absence of the β-pinene isomer of pinene.

Optionally, the use is the use of the (-)-α enantiomer of pinene and the (+)-α enantiomer of pinene in a ratio of about 500:1.

Optionally, the use is the use of the (-)-α enantiomer of pinene and the (+)-α enantiomer of pinene in a ratio of about 500:50.

The use may comprise the use of substantially optically pure (-) - α enantiomer of pinene for inducing or stimulating growth of keratinous fibres.

The use may comprise the use of substantially optically pure (-) - α enantiomer of pinene which is substantially in the absence of the (+)-α enantiomer of pinene.

Also described is a kit of parts comprising the composition and an applicator.

### Brief Description of the Figures

In the Figures, which illustrate the preferred embodiments of the invention by way of example only:
Figure 1 shows skin of a control mouse, 5 weeks after topical administration of acetone. Epidermis is thin and rare hair follicles can be seen in the dermis. H & E, 70x
Figure 2 shows mouse skin 5 weeks after topical administration of X dissolved in acetone. Moderate hyperplastic epidermis and very numerous, enlarged hair follicles in the dermis and hypodermis. Most hair follicles are in cross-sections. H &E 70x.
Figure 3 shows mouse skin 6 weeks after topical treatment of X dissolved in acetone. Longitudinal or oblique sections of numerous and enlarged hair follicles in the dermis and hypodermis. H&E 70x.
Figure 4 shows high magnification of the same mouse skin as seen in Figure 2. Cross sections of numerous, well differentiated hairs. E: hyperplastic interfollicular epidermis with acanthosis. Hyperkeratosis and parakeratosis. Arrow: sebaceous glands in the vicinity of hair follicles. H & E 400x
Figure 5 shows longitudinal sections of three well-differentiated hair follicles in mouse skin 6 weeks after topical treatment with the solution containing X. H&E 400x
Figure 6 shows skin of a Sham treated (acetone alone) rabbit during 5 weeks. Rare hair follicles are seen. H& E 70x
Figure 7 shows skin of rabbit after 5week treatment twice weekly with X. Note very numerous, extremely long hair follicles and hyperplasia of interfollicular epidermis clearly visible. H&E 70x.
Figure 8 shows skin of a guinea pig treated twice weekly with carrier (acetone) during 5 weeks. Only rarely one can find new hair follicles. H&E 70x.
Figure 9 shows skin of guinea pig treated twice weekly with x in acetone. Numerous, well differentiated hair follicles are noted along with numerous newly growing follicles. H&E stain, 70x.
Figure 10 shows skin of a rat treated twice weekly with acetone (Sham) during 5 weeks. Only a few, rare hair follicles can be found. H&E stain, 70x.
Figure 11 shows skin of rat treated twice weekly with X in acetone during 5 weeks. Numerous hyperplastic hair follicles can be found. H &E stain, 70x.
Figure 12 shows the average number of hair follicles per mm of skin area in the control mice treated with turpentine oil or (-) -α-pinene treatment.

### Detailed Description of the Preferred Embodiments

**Pinene** (1*S*,5*S*)-2,6,6-trimethylbicyclo[3.1.1]hept-2-ene or (1*S*,5*S*)-6,6-dimethyl-2-methylenebicyclo[3.1.1]heptane, has two structural isomers: α-pinene and β-pinene. α-pinene has enantiomers (-)-α-pinene, and (+)-α-pinene, as illustrated below:

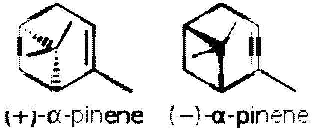

| | | | | |
|---|---|---|---|---|
| **name** | (1*R*)-(+)-α-pinene | (1*S*)-(-)-α-pinene | (1*R*)-(+)-β-pinene | (1*S*)-(-)-β-pinene |
| **CAS number** | 7785-70-8 | 7785-26-4 | 19902-08-0 | 18172-67-3 |

A mixture of pinene extract was able to induce hyperplasia of hair follicles after using a topical treatment, without any apparent local toxicity and impairment of cellular or tissue differentiation.

### Materials and Methods:

Young and adult male mice of NMRI strain weighing approximately 35g were fed basal diet A.O.3 supplied by UAR Villemoison-sur-Orge, France and water access *ad libitum.* All other mammals (rodents (mice, rats, guinea pigs and rabbits) were of the same origin.

In all experiments the same protocol was utilized unless specified:
Hairs were cut and trimmed carefully with electric clippers for an area of 6 cm² approximately in the sub scapular areas 2 days before treatment.

A dose of 0.1 ml of 50% acetone solution of pinene was topically administered twice weekly by dropping on the trimmed skin area.

Treatment lasted 5 week long. The hair growing agent pinene was of plant origin as used in most experiments as a crude, technical form.

Control Sham animals were treated with the solvent-carrier of pinene (usually acetone and in one experiment it was 80% ethanol) at the same dosage, or without Sham treatment at all when trimmed area was left without anything (absolute control).

Modifications of the protocol are described in the results and Tables.

All animals were sacrificed by decapitation and skin specimens were fixed in 10% formalin, embedded in paraffin, cut as 5 µm thick sections that were stained with hematoxylin and eosin (H&E). Both DNA and RNA detections were stained by methyl green-pyronine method. Acid DNAse activity was detected histochemically using lead citrate as a capturing contrast agent.

The effects of all treatments were semi-quantitatively evaluated by counting the number of hair follicles in an area of 1 mm long at a microscopic level of 100 x and were performed along 5 different areas of each histological sample and slides of each experiment The mean values +/- S.D. were calculated.

In the acute toxicological study weekly total body weights in groups of 12 mice were measured, following by terminal haematological examination, general autopsy, main organ weights and histological investigation of 7 mice per group were done. A group of treated mice with 50% acetone solution of pinene twice weekly during 5 weeks was compared with similarly treated mice with acetone and without any treatment

### Results:

### Treatment with pinene in 50% acetone:

A summary of the topical treatments using acetone 50% solution of pinene twice weekly during 5 weeks duration is summarized in Table I.

This treatment has produced in mouse skin at least a doubling of the mean number of hair follicles in a group of 5 mice when compared with Sham treated mice treated with acetone alone or with nothing at all (absolute control). Furthermore there were no differences in the number of hair follicles between these two control groups, suggesting that the solvent of acetone alone had no influence on the hair follicles' growth. In addition, those treatments did not cause any change in the integument normal histology.

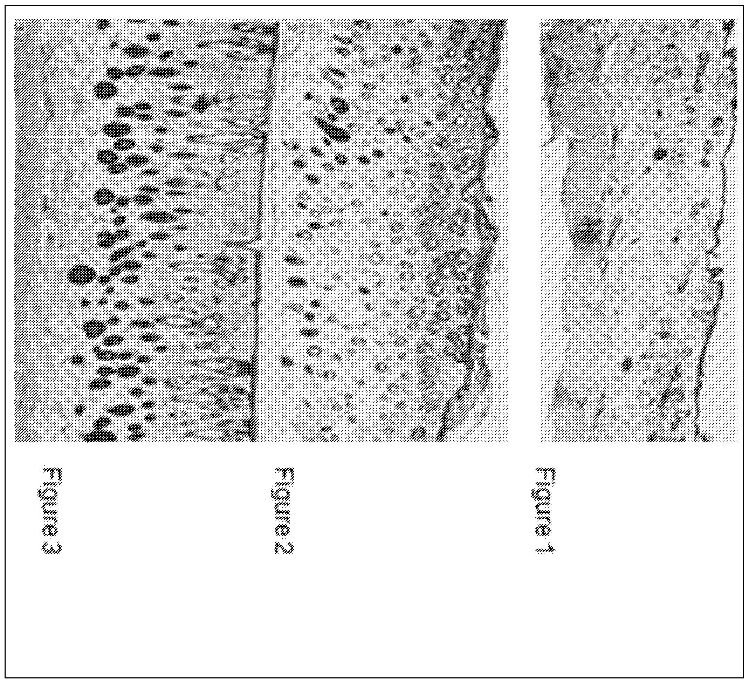

Examining the same treatment group one can note that the increased hair follicles grew much more than the mean values indicated in Table I (as shown in Figs 1 and 2). After 6 weeks of treatment no more increase of hair follicles was found (Fig. 3). It has to be noted here that in all the semi quantitative calculations reported each hair follicle visible in the examined area of the histological section was counted but it may have happened that a slight error of 2 or more cross-sections of the same follicle was counted as some tissue sections were not always perfectly perpendicular to the skin surface. However, the same error of counts could have happened in both treated and untreated samples, for the same reason.

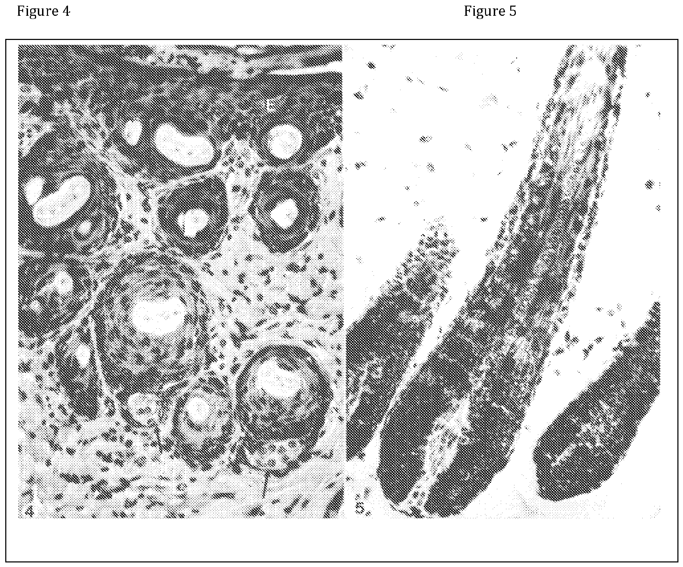

The aforementioned hyperplastic hair follicles in treated mouse skin (Figs 2 and 3) also demonstrated a hypertrophic pattern; this means that not only their number - but also their volume size was increased. They occupied the entire thickness of the dermal layer of the skin and their lower parts were located deeply in the hypodermis. Using higher magnification these hyperplastic and hypertrophic hair follicles appear on cross and longitudinal sections with a characteristic pattern of layers of well differentiated cells like in mature hair follicles of a normal skin of untreated mouse (Figs. 4 and 5).

One to three hairs per follicle can be found and close to the follicular body were well-developed sebaceous glands. As shown in Figure 5 the papillary bodies at the bottom of densely distributed hyperplastic hair follicles are also very well developed. Mitoses were relatively frequent but without any anomalies.

Histochemical staining for DNA and RNA of Brachet demonstrated a rich content. Similarly acid DNAse was intensely positive in the nuclei and in the cytoplasm as granule-like in normal mouse skin. In the treated mouse skin a moderate hyperplasia of the adjacent, interfollicular epidermis with signs of acanthosis, hyperkeratosis and sometime parakeratosis were observed (Fig 4). The dermal layer of the treated mouse was somewhat thicker than in control animals and contained a slight inflammatory exudate mainly lympho- and histiocytic. No vesicular or ulcerative change could be found.

### Decreased of carrier/solvent and use of ethanol results in the same hair follicles' hyperplasia as previously found:

It became interesting to know whether the concentration of pinene could be decreased and whether the solvent (acetone) could be replaced by others (such as ethanol) which would be less harmful and easier to use.

As shown in Table 2, the decreased concentration of pinene in acetone to 5% administered twice weekly, as well as 5% of pinene in 80% ethanol administered 5 times weekly practically did not decrease the hyperplastic actions of pinene on mouse hair follicles after 5 week treatment

### Other animals:

It was investigated whether the same treatment could produce the same results in other species of mammals. In that aim, a similar treatment with pinene as described in Methods was administered in rat, guinea pig and rabbit.

Table 3 shows that the number of hair follicles increases in the other animals with 50% acetone solution of pinene administered topically for 5-week duration. In these experiments, the animals were individually evaluated by calculating the percentage of increased hair follicles in treated areas compared with the untreated areas.

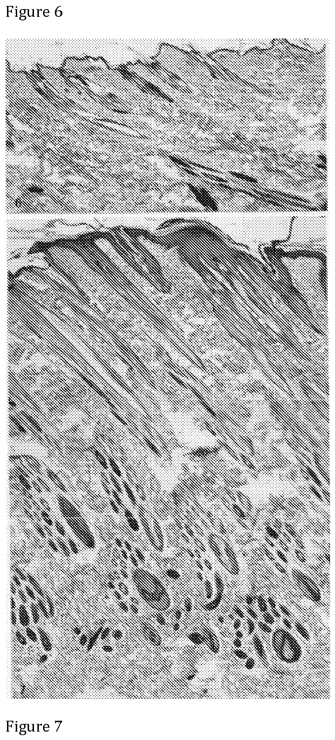

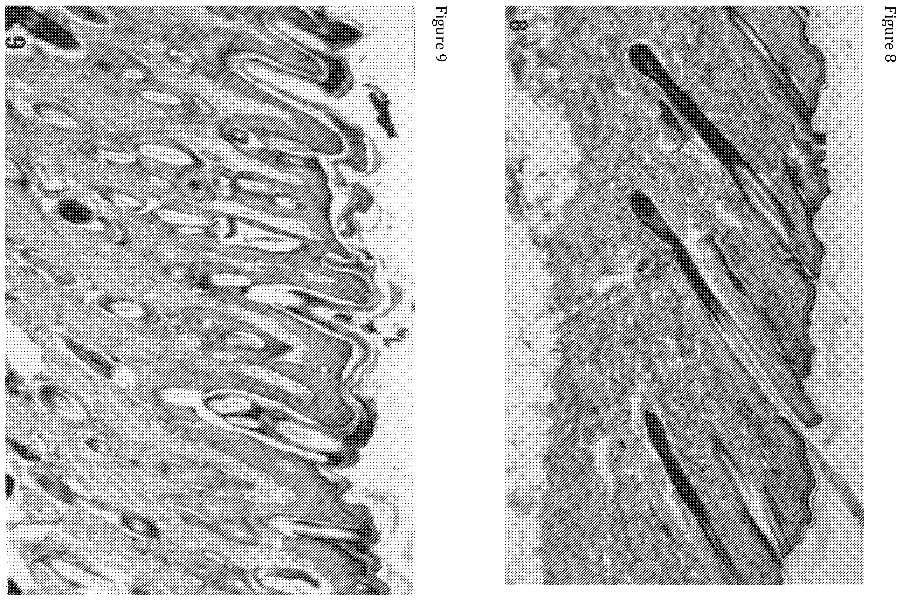

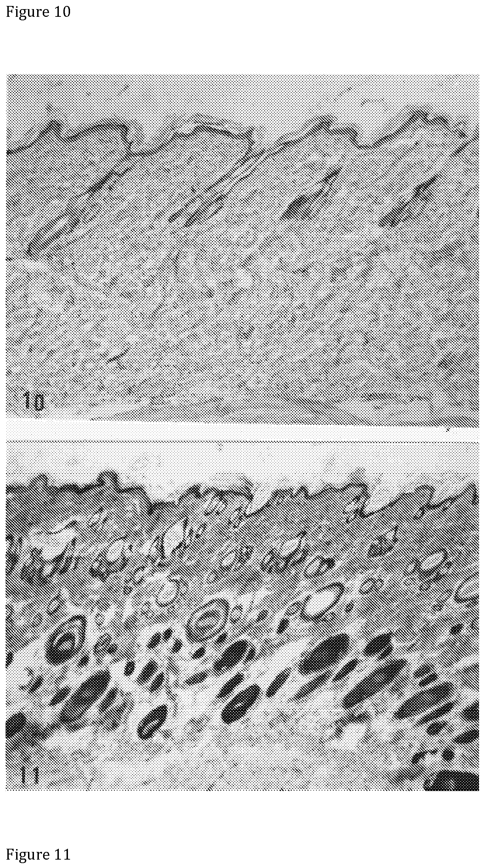

Table 3 shows that the increased follicles range from 80% to 300% from comparisons and the highest values were found in rat and the lowest in guinea pig. Histological pattern (Figs 6 to 11) of these hyperplastic hair follicles in rabbit, guinea pig and rat demonstrated the same pattern of good differentiation with no histopathologic changes as those observed in the treated mice (inflammatory and some minor integumentary pathology).

### Other integument samples taken to verify low or no toxicity of pinene and carrier:

In addition to the control animals treated with acetone alone as Sham or control groups, skin samples were taken at a distance of the area treated from all of the treated animals. All show that the pinene effects were essentially local, act at the treated area only because the histology did not show any hyperplastic effect in areas adjacent to treatment i.e. the actions of pinene are strictly localised without any diffusion to other areas of the integument The mean number counts of hair follicles in the untreated areas of the treated animals were similar to those found in control animals, treated solely by acetone or alcohol. These results concerned all examined species (mice, rat, guinea pig and rabbit).

### Persistence of action of pinene on hair follicles:

The hyperplastic action of the product pinene is similar to the HFRF action. In similar experiments one looked at the persistence of the hair growth and hyperplasia. The results in the same Table 3 demonstrated that 8 weeks after interruption of the 5-week treatments with X the induced hyperplasia was still present, although in a decreased fashion. For example, in rabbit the percentage of hair follicle counts in treated areas calculated against untreated skin areas decreased after 8 weeks from more than 100% to 29-55% and in guinea pig from 94% and 84% to 80% and 60% respectively (Table 3).

The mean number of hair follicles in mice decreased from 54.4% to 39.6% and 30.6% respectively after 2 and 5 weeks after the lag period interrupting the pinene treatment (Table 4).

### Time delay or Latency:

The latency to induce the hyperplastic effect in mice by standardized treatment of pinene is described in Methods and presented in Table 5. As shown in Table 5, the mean number of hair follicles started to increase after 2 weeks of treatment, reaching its maximum level after 4 weeks of treatment Later on, the number of follicles remained quite stable.

### Pinene extract:

It was investigated whether agents of the same origin but extracted or produced in different manner (e.g. partially purified) or originating from a different but similar species of plant have the same effects on hair follicles.

The results of the experiments are presented in Table 6. Similar hyperplastic effects on hair follicles in mice were induced by pinene of technical origin, by the rectified product of the same origin, as well as by a product originating from different but similar plant species.

### Toxicology tests:

Tables 7 to 9 summarize the results of acute toxicology tests of 50% acetone solution of pinene topically administered twice weekly during 5 weeks in mice.

As one can see in these Tables, no significant change concerning the total body weight, haematological examination and main organ weight have been noted in the group of treated animals compared with that of Sham treated or being without any treatment or absolute control group.

In these animals, no clinical signs or significant changes in general autopsy were revealed macroscopically. Histological slides of main organs did not permit to detect any alterations or abnormal morphology. The histologic investigations of hematoxylin-eosin stained slides of organs from 7 mice per group included the following organs: liver, lungs, heart, thymus, spleen, kidneys, small and large intestine, pancreas, adrenals, testicles, brain and brain stem.

### Discussion:

50% acetone solution of pinene topically administered twice weekly during 5 weeks promoted and induced more than a doubling of the number of hair follicles in mice.

These hyperplastic and hypertrophic hair growths had a pattern of mature, well differentiated follicles containing 1 to 3 hairs per follicle. They did not demonstrate any feature of 'delayed cellular maturation' or of the 'reversal of cell differentiation' both alterations being characteristic effects of known skin tumour promoters. Moreover the cells of these follicles presented normal activity of acid DNase, enzyme which becomes deficient after the action of known tumour promoters and during the early stages of carcinogenesis.

The large amount of DNA and RNA in the regions studied appear normal and in the case of tumour cells it would increase, being decreased or lacking in degenerated cells as well as in an early stage of cell necrosis.

All these features seemed to demonstrate that the above described hair follicle proliferations are accompanied by normal cell maturation, and those are devoid of degenerative or necrotizing events and certainly are not accompanied by tumour promoting tendencies. Obviously to exclude with certainty carcinogenic possibility a chronic dosage experiment should be tested.

There was no hyperplasia of hair in the untreated areas (absolute control zones) as well as the absence of any clinical and morphological pathology in the acute toxicology tests of chronic treatment The tests investigated also suggested an absence of carcinogenicity and mutagenicity, and weak allergenic effect.

### Rapidity of action:

The induction of the hair follicle hyperplasia was quite rapid. It started after 2 weeks of topical treatment and persisted relatively well after interruption of the treatment for 2 to 8 weeks, obviously with a tendency to decrease with time.

### Results

**Table 1**

| | |
|---|---|
| The effect of local treatment during 5 weeks (twice weekly) by 5% pinene dissolved in 50% acetone and the number of hair follicles in mice compared with the group of mice only treated with the carrier-solvent acetone (Sham) or being without treatment (absolute control). | |

| **Groups** | Mean number of hair follicles per mm of skin in 5 mice +/- SD |
|---|---|
| Absolute control | 17.72 +/- 6.39 |
| 50% Acetone | 17.36 +/- 6.49 |
| 50% acetone solvent + 5% pinene | **41.48 +/- 11.05** |

**Table 2**

| | | |
|---|---|---|
| The effect of different dosing and different solvents of pinene topically administered weekly during 5 weeks on the number of hair follicles observed in shaved area in mice | | |

| **Treatments** | Nb of mice | Mean number of hair follicles per 1 mm of skin +/- SD |
|---|---|---|
| Absolute control | 5 | 17.72 +/- 6.39 |
| 50% of acetone solution of pinene twice weekly | 5 | 41.48 +/- 6.49 |
| 5% of pinene in 50% acetone solvent twice weekly | 7 | 42.20 +/- 11.61 |
| 5% of pinene in 80% ethanol solvent 5 times weekly | 10 | 39.82 +/- 9.87 |

**Table 3**

| | | | | | |
|---|---|---|---|---|---|
| **The average, mean number of hair follicles per 1 mm of treated skin and Sham skin areas** with pinene dissolved in 50% acetone twice weekly during 5 weeks in rabbits, guinea pigs, rats and mice compared to Control animals treated solely with acetone. In some groups the persistent hyperplastic effect was examined 8 weeks after treatment. | | | | | |

| Species / Treatment | Mean number of hair follicles per mm2 of skin directly after treatment | | % of A against B | C Mean number of hair follicles per 1 mm of skin 8 weeks after treatment | % of C Against B |
|---|---|---|---|---|---|
| | A Treated Area | B Untreated area | | | |
| Rabbit / Control | 22 | 27.8 | -20.86 | 21.4 | -23.02 |
| Rabbit / Control | 20 | 23.4 | -14.53 | 18.4 | -21.02 |
| Rabbit/Treated | 45.6 | 25.6 | 78.19 | 36.6 | 42.97 |
| Rabbit/Treated | 56.4 | 23.8 | 136.97 | 37 | 55.46 |
| Rabbit/Treated | 60 | 20.8 | 188.46 | 31.4 | 50.96 |
| Rabbit/Treated | 59.6 | 25.4 | 104.65 | 32.8 | 29.19 |
| | | | | | |
| Guinea Pig/ | 19 | 23 | -17.39 | 20.2 | -12.17 |
| Control | 10 | 13.6 | -26.41 | 12.4 | -8.82 |
| Guinea Pig/ | 32 | 16.4 | 95.12 | 29.6 | 80.49 |
| Control | 33.6 | 18.2 | 84.62 | 29.2 | 60.44 |
| Guinea Pig/ | | | | | |
| Treated | 13 | 9.6 | 56.25 | - | - |
| Guinea Pig/ | 15.6 | 13.2 | 25.76 | | |
| Treated | 43.8 | 11.6 | 277.59 | | |
| | 34 | 7.8 | 335.90 | | |
| Rat /Control | 38.4 | 9.6 | 300.00 | | |
| Rat / Control | | | | | |
| Rat/Treated | 17.6 | 18.4 | -4.34 | - | - |
| Rat/Treated | 14.2 | 14.2 | 0 | | |
| Rat/ Treated | 40.6 54.2 | 21.2 17 | 91.50 218.82 | | |
| Mouse / Control | 47.6 | 22.2 | 114.41 | | |
| Mouse / Control | 47.8 | 17 | 181.17 | | |
| Mouse/ Control | 41.2 | 20.2 | 103.96 | | |
| Mouse / Treated | | | | | |
| Mouse / Treated | | | | | |
| Mouse / Treated | | | | | |
| Mouse/ Treated | | | | | |

**Table 4**

| | |
|---|---|
| **The mean number of hair follicles +/- S.D. per 1 mm of skin slide** in different groups of mice directly after 5 weeks of treatment (twice weekly) with pinene dissolved in 50% acetone and after intervals of 2 or 5 weeks without treatment The results are also compared to Absolute control. | |

| **Groups** | Mean number of hair follicles +/- S.D. per 1 mm of skin slide |
|---|---|
| Absolute control (5 mice) | 17.72 +/- 6.39 |
| Directly after treatment (7 mice) | 54.43 +/- 12.23 |
| 2 weeks after treatment (5 mice) | 39.60 +/- 11.68 |
| 5 weeks after treatment (4 mice) | 30.60 +/- 10.40 |

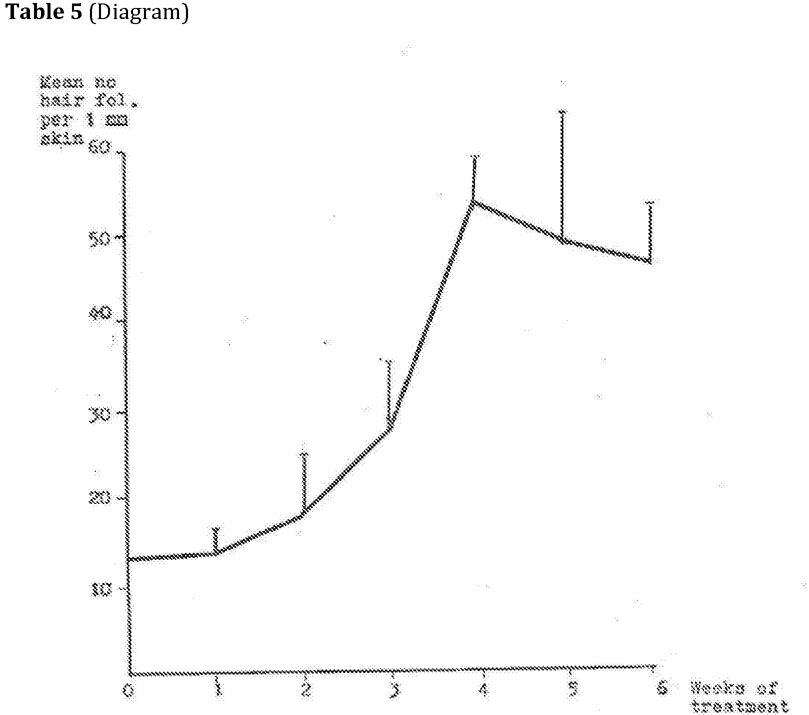

**Table 6**

| | |
|---|---|
| **Mean number of hair follicles +/- S.D. per 1 mm of skin slide** in 7 mice per group after 5 weeks of tteatment (twice weekly) with pinene dissoved in 50% acetone from different sources (technical or rectified from the same plant origin and one from another but similar species of plant). The results are compared with the group of mice treated solely with acetone. | |

| **Treatments** | **Mean number of hair follicles +/- S.D. Of skin slide** |
|---|---|
| **Acetone (Control)** | **21.89 +/- 7.96** |
| **Pinene technical** | **54.43 +/- 12.29** |
| **Pinene rectified** | **52.49 +/- 12.04** |
| **Pinene from another Plant species** | **42.40 +/- 9.83** |

**Table 7**

| | | | | | | |
|---|---|---|---|---|---|---|
| Mean total body weight +/- SD weekly measured in groups of 12 mice 5 weeks topically treated (twice weekly) with 50% of acetone solution of pinene or acetone alone (Sham), compared with a group of 10 mice without any treatment (absolute control) | | | | | | |

| **Treatments** | **Mean total body weight** +/- SD at consecutive weeks of treatment | | | | | |
|---|---|---|---|---|---|---|
| | 0 | 1-week | 2-week | 3-week | 4-week | 5-week |
| Absolute Control | 34.87+/- 1.4 | 36.85+/- 1.2 | 37.50 +/- 1.2 | 37.45+/- 1.8 | 38.25+/- 1.8 | 38.89+/- 1.6 |
| 50% Acetone (Sham) | 34.88+/- 1.4 | 36.71+/- 1.2 | 37.23 +/-1.5 | 37.71+/- 1.9 | 38.14+/- 2.1 | 38.99+/- 1.5 |
| 5% pinene in 50% acetone | 36.07+/- 1.6 | 37.98+/- 2.5 | 37.87+/- 1.9 | 37.73+/- 2.1 | 37.88+/- 2.2 | 38.48+/- 1.7 |

**Table 8**

| | | | | | | | |
|---|---|---|---|---|---|---|---|
| **Results of haematological examination** in a group of 7 male mice treated twice weekly topically with pinene dissolved in 50% acetone during 5 weeks compared to 7 control Sham, male mice treated 5 weeks with acetone alone | | | | | | | |

| Treatment | Mean number of blood cells +/- SD | | Mean % of blood cells +/- SD | | | | |
|---|---|---|---|---|---|---|---|
| | Erythro | White | Lymphocytes | PMN | Eosinophils | Basophils | Monocytes |
| Acetone | 4,857,143 +/- 745782 | 12,000 +/- 3014 | 53.29 +/- 6.2 | 38.0 +/- 6.8 | 2.0 +/- 0.8 | 0.71 +/- 0.9 | 6.29 +/- 3.8 |
| Pinene in Acetone | 4,685,715 +/- 681734 | 7,928 +/- 975.9 | 56.29 +/- 9.1 | 32.8 +/- 1.4 | 1.86 +/- 0.7 | 0.57 +/- 0.7 | 7.57 +/- 1.4 |

**Table 9**

| | | | | | | |
|---|---|---|---|---|---|---|
| **Mean total body weight and mean organ weight +/-SD** per group of 7 male mice after 5 weeks of twice weekly topical treatment of pinene dissolved in 50% acetone versus 7 male mice Sham treated (by acetone alone) and also 7 male mice untreated or Absolute control (5 mice). | | | | | | |

| Mice groups as treated | Total body weight gram +/- SD | Organ weight in mg +/- SD | | | | |
|---|---|---|---|---|---|---|
| | | Liver | Spleen | Brain and stem | 2 kidneys | 2 testicles |
| Absolute control | 38.7 +/-1.57 | 1946.4 +/- 48.79 | 174.3 +/- 8.94 | 472.0 +/- 22.8 | 576.9 +/- 29.66 | 229.8 +/- 11.40 |
| Acetone alone | 38.7 +/- 1.38 | 2011.4 +/-111.12 | 197.1 +/- 16.04 | 459.2 +/- 7.32 | 611.4 +/- 53.98 | 228.5 +/- 32.37 |
| Pinene in Acetone | 37.7 +/- 1.20 | 1910.0 +/- 165.13 | 164.2 +/- 26.37 | 164.2 +/- 26.37 | 594.2 +/- 43.93 | 251.4 +/-21.16 |

### Identification of the most active molecule that can induce an activation and an increased number of hair follicles in a treated animal.

Turpentine oil was noted to have induced an increased number of mature hair follicles in 4 different animal species is composed of several well-known organic compounds.

Pinene contains the following main compounds:

| | |
|---|---|
| 60 - 96% | α-pinene |
| 0.6 - 28% | β -pinene |
| 1.0 - 3.7 % | dipentene |
| 0.9 - 1.7 % | camphene |
| 0 24% | Δ³ -carene |
| 0 - 2.8 % | p-cymol |

and other compounds as about 0.7 - 4.1 %

According to the conditions of production and conservation of the turpentine oil, different products of oxidative decomposition can appear. In addition, the composition of turpentine oil also depends on the species of plant and its geographical origin.

All turpentine oils contain high proportions of α-pinene, but the amounts of Δ 3 -carene may be very different from one product to another. For example, the turpentine oils originating from Northern regions of Europe (Sweden, Finland, Russia) about 30 to 40 % of Δ³-carene can be present - whereas in turpentine oils from Southern Europe (e.g. France, Portugal, Spain) and from the United States, there are very low to negligible amounts of this compound. It should be underlined that it is the presence of this organic molecule or its hydroperoxide that induces a high allergenic and toxic properties of the turpentine oil which can be dangerous for humans.

Instead of using a mixture of components of a crude extract from plant origin, a single component has been surprisingly identified as a specific molecule having an action to assist and induce in the growth and multiplication of the hair follicles.

This study investigating such capability was undertaken with two possibilities in mind:
(1) to isolate the components from a crude extract product of the turpentine oil and to investigate separately their respective activities on the skin of experimental animals
(2) to utilize commercially available known compounds of turpentine oil compound on a similar animal experiment

The investigation included research into the most abundant compounds found in turpentine oil, i.e. the α-pinene and β-pinene and if available separately, using their chirality and investigating both the dextrogyre and levogyre forms.

### Materials and Methods (II)

The following commercially available, components of turpentine oil were investigated:
(-) α-pinene; product No 13.127.32 Janssen Chim. 2440 Geel, Belgium
(+) α-pinene; product No 13.126.31 Janssen Chim. 2440, Geel Belgium
(-) β-pinene; product No 15.032.94 Janssen Chim, Geel, Belgium
Turpentine oil crude industrial product was originated from Portugal.

Young adult, male mice of the N.M.R.I. strain, weighing at the beginning of the experiment +/- 30 g were used, as in previous experiments.

The protocols used were similar to those described in the previous experiments and study was realized during summer months (July and August). Hair coats were cut along the dorsal surfaces with electric clippers in an area of +/- 6.0 cm² two days before treatments. Mice were divided into 4 groups of 10 animals. Each mouse received either
(1) A dose of 0.1 mL of 5% absolute ethanol solution of turpentine oil,
(2) (-) α-pinene,
(3) (+) α-pinene or
(4) (-) β-pinene administered topically on the surface of the shaved skin twice weekly.

Control groups of the same number and similar age and sex mice were treated the same way but topically applied the solvent alone (absolute ethanol).

After a 4-week treatment duration all the mice were sacrificed by decapitation, and general autopsies were performed in which skin and main organs specimens were excised and fixed in 10% formalin for histological examination after paraffin embedding and hematoxylin -eosin staining.

The following evaluating criteria of the observations and results were used:
(1) body and main organ weights,
(2) skin and main organs histological patterns,
(3) semi quantitative evaluation of hair follicles per 1 mm long area in a 5 different areas of the histological sections of the skin of each animal in each group at 100 x microscopic magnification.

### Results

During the entire period of the experiment, no pathological symptoms in any animal were observed.

Their total body weights did not present any significant differences when compared with the control animals (Table 10).

General autopsies have not revealed any macroscopically detectable alterations of the skin and of the many organs examined, including their weights, compared to those of the control mice (Table 10).

Histological examination of liver, kidneys, testicles, thymus, and brain did not show microscopic lesion.

### Hair follicle growths:

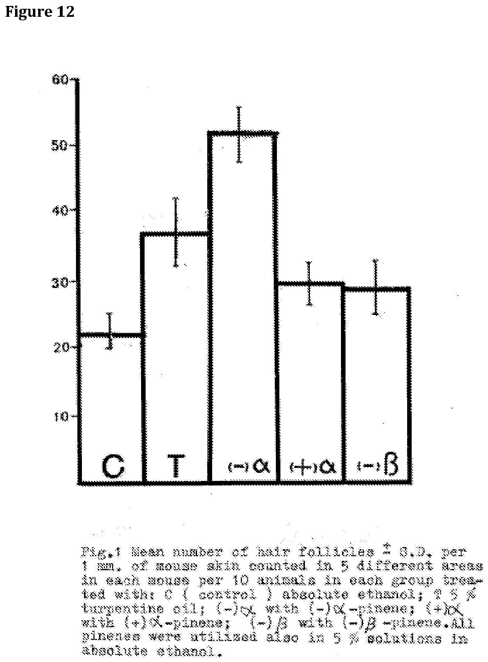

Table 11 and Figure 12 represent the average number of hair follicles per mm of skin area in the control mice.
**1. With turpentine oil treatment** 37.3 hair follicles were counted, versus control 22.3.
**2. Following (-) α-pinene treatment** those mice had 52.6 hair follicles, more than a doubling of those of the control group and significantly more than the group treated with the same concentration of turpentine oil. In this group, 6 out of 10 mice had more than 50 hair follicles.
**3.** Surprisingly, the mice treated with the same concentration of (+) α-pinene as well as with β-pinene only demonstrated a slightly increased number of hair follicles if compared with the control animals: 28.9 after (+) α-pinene treatment versus 28.1 after β-pinene treatment
**4. After 4 weeks of treatment with 5% solution of the (-) α-pinene** the histological pattern considerably increased the number of hair follicles and was almost as typical as in mature hairs, containing 1 to 3 hairs and often with their associated and adjacent sebaceous glands.

The epidermis of these mice was slightly hyperplastic with 3-5 layers in some areas instead of normally existing 2-3 layers. Any sign of the delay of maturation as multiple layers of undifferentiated basal cells was not observed. There was no sign of metaplasia or neoplasia such as exaggerated acanthosis or keratosis. Only in rare cases there were some insignificant dermal infiltrates of a mononuclear type without any polynuclear leucocytes, fibrosis, necrosis or epidermal ulceration, which would indicate inflammation or early stage of inflammation.

The results obtained in the above paragraphs of the investigations can be summarized as follows:
1. (-) α-pinene in the most active molecule, inducing hair follicles multiplication amongst the 3 principal compounds of turpentine oil examined. Its activity is more than double of that of in the control group.
2. (-) α-pinene is more effective than the crude product of turpentine oil at the same concentration and in the same solvent
3. (-) α-pinene is active at relatively low dose (up to 5%) in a harmless solvent (ethanol) commonly utilised in human dermatology or cosmetology.
4. Both (+) α-pinene and (-) β-pinene are at the same dose and in the same solvent distinctly less capable than (-) α-pinene. Their activity is only slightly superior to the effects of treatment with the solvent lone in control animals.
5. Both (+) α-pinene and (-) β-pinene are also less active than turpentine oil at the same dose.
6. The most effective compound, (-) α-pinene did not produce any local skin alteration detectable by clinical signs or microscopic examination. There was neither any sign of a possible general toxicity.

The utilisation of one specific molecule, i.e. (-) α-pinene in the future animal and human application has numerous advantages:
1. Its effectiveness is greater than that of turpentine oil
2. Its toxicity and/or allergenic aspect are lower or not existent after elimination of the Δ³-carene.
3. It can facilitate the elaboration of the best galenic form to be used and to develop the most efficient clinical protocol, if it means necessary to be use clinically.
4. (-) α-pinene could be used to stimulate growth of hair follicles and could work without need of transplanting individual grafting of hair follicles in individual who may respond favourably with the compound treatment
5. The compound does not have to be taken orally, like other drugs favouring hair growth, which eliminates some internal, dangerous toxicology tests.

The composition for use in inducing or stimulating growth of keratinous fibres according to the present invention comprises (-) α-pinene as an active ingredient. The concentration of α-pinene is preferably limited to 5% in the composition, so as to minimise toxicity risks. However, the concentration of α-pinene in the composition could be increased for greater strength up to a limit of 10% w/w. The addition of a fragrance such as rosemary or verbinol to the composition would increase the overall concentration, since the oils of these fragrances also contain some pinene.

The (+) -α-pinene enantiomer has been demonstrated to provide anti-inflammatory and antibacterial effects at very low concentrations. Therefore it would be desirable in some embodiments of the invention to include a low concentration of (+) -α-pinene in the composition. This would provide a synergistic effect on inducing or stimulating growth of keratinous fibres, since the (+) -α-pinene would prevent inflammation or infection of sebaceous glands required for optimum growth. Even a very small amount of (+) -α-pinene could provide the synergistic effect, such as at a concentration in the composition of around 0.01% to around 1%. If desired, the concentration of pinene could be greater, such as up to 10%.

**Table 10:**

| **Mean total body weight and main organs'weights +/- S.D. for 10 mice per group in 5 differently treated groups of animals.** | | | | | |
|---|---|---|---|---|---|
| **All values are Mean +/- S.D.** | **Control (Ethanol)** | **5% Turpentine oil** | **5% (-) α-pinene** | **5% (+) α-pinene** | **5% (-) β-pinene** |
| **Total body weight in gram** | 40.3 ± 2.05 | 41.2 ± 4.15 | 39.5 ± .42 | 40.1 ± 3.73 | 37.0 ± 4.48 |
| **Liver** | 2872 ± 273.9 | 2510 ± 248.0 | 2790 ± 449.9 | 2834 ± 283.4 | 2504 ± 301.3 |
| **Spleen** | 330 ± 23.4 | 376 ± 59.4 | 332 ± 24.9 | 390 ± 36.7 | 350 ± 36.7 |
| **2 kidneys** | 890 ± 102.9 | 822 ± 21.6 | 814 ± 121.7 | 840 ± 89.7 | 797 ± 23.3 |
| **2 testicles** | 444 ± 23 | 410 ± 21.2 | 440 ± 41.8 | 432 ± 34.9 | 444 ± 41.5 |
| **Thymus** | 254 ± 23 | 284 ± 23 | 252 ± 22.8 | 266 ± 18.1 | 244 ± 9.0 |
| **Brain** | 654 ± 11.4 | 634 ± 24 | 674 ± 23 | 658 ± 23.8 | 660 ± 12.2 |

**Table 11:**

| **Mean number of hair follicles +/- S.D. per 1 mm of skin -slide counted in 5 different areas in each animal of 5 groups of 10 mice** | |
|---|---|
| **Treatments** | **Mean number of hair follicles per 1 mm of skin +/- S.D.** |
| **Control (Absolute ethanol)** | **22.5 ± 4.5** |
| **5% Turpentine oil** | **37.5 ± 11.3** |
| **5% (-) α- pinene** | **52.6 ± 7.8** |
| **5% (+) α- pinene** | **28.9 ± 6.4** |
| **5% (-) β- pinene** | **28.1 ± 7.6** |

The effective dose of pinene can be decreased (as low as 5%) and 80% ethanol can be utilized as its solvent instead of acetone. It was also found that a partially or substantially purified pinene was similarly active as the crude turpentine product (i.e. without dipentene, camphene, Δ³-carene and p-cymol).

Induction of hair follicle hyperplasia in 4 different animal species by pinene support the possibility of obtaining the same results in wool producing animals (e.g. sheep) and this topical treatment may have an application and importance for agricultural and economic development The same argument reinforces the suggestion of using the pinene preparation for treating human baldness in adult males or caused by anticancer treatments.

Comparing data with both 5% alpha (+) pinene and β (beta) pinenes it is alpha (-) pinene that is the most active. Pinenes were purchased as liquids, flammable at 98% concentration from Janssens Pharmaceutica, as 500 ml bottles.

## Claims

1. A composition for a therapeutic use in inducing or stimulating growth of keratinous fibres for treating human baldness in adult males or caused by anticancer treatments, wherein the composition comprises pinene as an active ingredient and wherein the active ingredient pinene comprises the α-pinene isomer of pinene and wherein at least 70% of the active ingredient pinene is the (-)- α enantiomer of pinene,
**characterised in that** the active ingredient pinene is present in the composition at between 0.5% to 10% (w/w).

2. The composition for use according to claim 1, wherein the active ingredient pinene is present as optically pure (-)- α enantiomer of pinene.

3. The composition for use according to any preceding claim, wherein the composition is free of the β-pinene isomer of pinene.

4. The composition for use according to any of claims 1-3, wherein the composition comprises the (-)-α enantiomer of pinene and the (+)-α enantiomer of pinene in a ratio of 500:1.

5. The composition for use according to any preceding claim, further comprising a solvent.

6. The composition for use according to any preceding claim, wherein the keratinous fibres comprise hair, wool, pelt, coat or fur.

7. The composition for use according to any preceding claim, wherein the composition is a cosmetic or a dermatological composition in the form of a shampoo, lotion, cream, gel, hair styling product, beauty product, skin preparation, pharmaceutical preparation or ointment.

8. The composition for use according to any preceding claim, further comprising a pharmaceutically or a cosmetically acceptable excipient.

9. Non-therapeutic use of the composition of any of claims 1 to 8 for increasing the density of the keratinous fibres.

10. A non-therapeutic method for inducing or stimulating growth of keratinous fibres comprising the step of administering to a subject an effective amount of a composition comprising pinene as an active ingredient as defined in any one of claims 1 to 4.

11. The method of claim 10, wherein the step of administering comprises topical administration of the composition.

12. The method of claim 11, wherein administration is to the scalp and/or hair of a human subject.

13. The method of any of claims 10 to 12, wherein the composition is administered twice weekly for at least 5 weeks.

## Patentansprüche

1. Eine Zusammensetzung für eine therapeutische Verwendung zum Induzieren oder Stimulieren des Wuchses von Keratinfasern zur Behandlung von menschlicher Kahlheit bei erwachsenen Männern oder verursacht durch Antikrebsbehandlungen,
wobei die Zusammensetzung Pinen als Wirkstoff beinhaltet und wobei der Wirkstoff Pinen das α-Pinen-Isomer von Pinen beinhaltet und wobei mindestens 70 % des Wirkstoffs Pinen das (-)-α-Enantiomer von Pinen ist,
**dadurch gekennzeichnet, dass** der Wirkstoff Pinen in der Zusammensetzung mit zwischen 0,5 % und 10 % (w/w) vorhanden ist.

2. Zusammensetzung zur Verwendung gemäß Anspruch 1, wobei der Wirkstoff Pinen als optisch reines (-)-α-Enantiomer von Pinen vorhanden ist.

3. Zusammensetzung zur Verwendung gemäß einem der vorhergehenden Ansprüche, wobei die Zusammensetzung frei von dem β-Pinen-Isomer von Pinen ist.

4. Zusammensetzung zur Verwendung gemäß einem der Ansprüche 1-3, wobei die Zusammensetzung das (-)-α-Enantiomer von Pinen und das (+)-α-Enantiomer von Pinen in einem Verhältnis von 500 : 1 beinhaltet.

5. Zusammensetzung zur Verwendung gemäß einem der vorhergehenden Ansprüche, ferner beinhaltend ein Lösungsmittel.

6. Zusammensetzung zur Verwendung gemäß einem der vorhergehenden Ansprüche, wobei die Keratinfasern Haar, Wolle, Pelz, eine Haardecke oder Fell beinhalten.

7. Zusammensetzung zur Verwendung gemäß einem der vorhergehenden Ansprüche, wobei die Zusammensetzung eine kosmetische oder eine dermatologische Zusammensetzung in Form eines Shampoos, einer Lotion, einer Creme, eines Gels, eines Haarstylingprodukts, eines Schönheitsprodukts, eines Hautpräparats, eines pharmazeutischen Präparats oder einer Salbe ist.

8. Zusammensetzung zur Verwendung gemäß einem der vorhergehenden Ansprüche, ferner beinhaltend einen pharmazeutisch oder einen kosmetisch akzeptablen Hilfsstoff.

9. Eine nichttherapeutische Verwendung der Zusammensetzung gemäß einem der Ansprüche 1 bis 8 zur Erhöhung der Dichte der Keratinfasern.

10. Ein nichttherapeutisches Verfahren zum Induzieren oder Stimulieren des Wuchses von Keratinfasern, das den Schritt beinhaltet, einem Individuum eine wirksame Menge einer Zusammensetzung zu applizieren, die Pinen als Wirkstoff wie in einem der Ansprüche 1 bis 4 definiert beinhaltet.

11. Verfahren gemäß Anspruch 10, wobei der Schritt des Applizierens die topische Applizierung der Zusammensetzung beinhaltet.

12. Verfahren gemäß Anspruch 11, wobei die Applizierung auf die Kopfhaut und/oder das Haar eines menschlichen Individuums erfolgt.

13. Verfahren gemäß einem der Ansprüche 10 bis 12, wobei die Zusammensetzung mindestens 5 Wochen lang zweimal wöchentlich appliziert wird.

## Revendications

1. Une composition pour une utilisation thérapeutique pour induire ou stimuler la croissance de fibres kératiniques pour traiter la calvitie humaine chez les mâles adultes ou provoquée par des traitements anticancéreux,
la composition comprenant du pinène en tant que principe actif et le principe actif pinène comprenant l'isomère α-pinène du pinène et au moins 70 % du principe actif pinène étant l'énantiomère (-) α du pinène,
**caractérisée en ce que** le principe actif pinène est présent dans la composition dans une quantité comprise entre 0,5 % et 10 % (p/p).

2. La composition pour une utilisation selon la revendication 1, dans laquelle le principe actif pinène est présent en tant qu'énantiomère (-) α optiquement pur du pinène.

3. La composition pour une utilisation selon n'importe quelle revendication précédente, la composition étant dépourvue de l'isomère β-pinène du pinène.

4. La composition pour une utilisation selon n'importe lesquelles des revendications 1 à 3, la composition comprenant l'énantiomère (-) α du pinène et l'énantiomère (+) α du pinène dans un rapport de 500/1.

5. La composition pour une utilisation selon n'importe quelle revendication précédente, comprenant en sus un solvant.

6. La composition pour une utilisation selon n'importe quelle revendication précédente, dans laquelle les fibres kératiniques comprennent des cheveux, de la laine, de la peau, du pelage ou de la fourrure.

7. La composition pour une utilisation selon n'importe quelle revendication précédente, la composition étant une composition cosmétique ou dermatologique sous forme de shampoing, de lotion, de crème, de gel, de produit de coiffure, de produit de beauté, de préparation cutanée, de préparation pharmaceutique ou de pommade.

8. La composition pour une utilisation selon n'importe quelle revendication précédente, comprenant en sus un excipient pharmaceutiquement ou cosmétiquement acceptable.

9. Utilisation non-thérapeutique de la composition de n'importe lesquelles des revendications 1 à 8 pour accroître la densité des fibres kératiniques.

10. Une méthode non-thérapeutique pour induire ou stimuler la croissance de fibres kératiniques comprenant l'étape consistant à administrer à un sujet une quantité efficace d'une composition comprenant du pinène en tant que principe actif telle que définie dans l'une quelconque des revendications 1 à 4.

11. La méthode de la revendication 10, dans laquelle l'étape consistant à administrer comprend l'administration topique de la composition.

12. La méthode de la revendication 11, dans laquelle l'administration se fait sur le cuir chevelu et/ou les cheveux d'un sujet humain.

13. La méthode de n'importe lesquelles des revendications 10 à 12, dans laquelle la composition est administrée deux fois par semaine pendant au moins 5 semaines.
